# EUROPEAN PATENT APPLICATION

(11) **EP 3 238 768 A1**
(43) Date of publication of application: **01.11.2017**
(21) Application number: 16167328.0
(22) Date of filing: 27.04.2016
(51) Int. Cl.: A61M 16/10

(54) **SYSTEM AND METHOD FOR DELIVERING OXYGEN AND PREVENTING HYPERCAPNIA**

(71) Applicant: Linde Aktiengesellschaft, 80331 München (DE)
(72) Inventor: Häußermann, Sabine, 82069 Neuried (DE); Bause, Matthias, 82205 Gilching (DE); Schmehl, Wolfgang, 82031 Grünwald (DE)
(74) Representative: m patent group

(57) **Abstract**

A system (100) for delivering oxygen to, and preventing hypercapnic respiratory failure in, a patient (1) is provided, the system (100) comprising an oxygen dosing unit (10) and a sensor arrangement (40), the oxygen dosing unit (10) being adapted to provide an oxygen flow (12) to a port (13) fluidly connectable to an oxygen delivery unit (30) attached to the patient (1), and the oxygen dosing unit (10) further being adapted to regulate parameters of the oxygen flow (12) on the basis of a first input signal indicating an oxygen status of the patient (1) and a second input signal indicating a carbon dioxide status of the patient (1). According to the invention, the sensor arrangement (40) comprises sensing means (42, 43) adapted to obtain a first sensor value corresponding to the oxygen status via a non-invasive transcutaneous measurement and a second sensor value corresponding to the carbon dioxide status via a non-invasive transcutaneous measurement, the sensor arrangement (40) being adapted to provide the first and second input signals on the basis of the first and second sensor values, and the oxygen dosing unit (10) comprising a control unit (11) programmed with an algorithm adapted to calculate the parameters of the oxygen flow (12) on the basis of the first and second input values and at least one further patient parameter relating to the patient (1). A corresponding method is also part of the present invention.

## Description

The present invention relates to a system for delivering oxygen to, and for preventing hypercapnic respiratory failure in, a patient, and to a corresponding method according to the pre-characterising clauses of the independent claims.

### Prior art

Excessive oxygen administration may lead to hypercapnic respiratory failure in some chronic obstructive pulmonary disease (COPD) patients. This is caused, as presently understood, by a ventilation-perfusion (Va/Q) mismatch and by the Haldane effect. For details, reference is made to the literature, e.g. W.F. Abdo and L.M.A. Heus, "Oxygen-induced hypercapnia in COPD: myths and facts", Crit. Care 2012, 16(5), 323, and D. Lynes, "Managing hypoxia and hypercapnia", Nurs. Times 2003, 99(11), 57.

For most COPD patients, a saturation of arterial oxygen (SaO₂) of 88 to 92%, compared with 94 to 98% for patients not at risk of hypercapnic respiratory failure, should be aimed at. However, considering values like SaO₂ or the partial pressure of arterial oxygen (PaO₂) alone may not be sufficient for long-duration oxygen treatment which is indicated in some COPD patients to avoid severe consequences of hypoxia. Over time, carbon dioxide elimination in such patients can vary, allowing for a higher or lower oxygen dosage to be tolerated without the risk of hypercapnic respiratory failure. Obviously, as long as hypercapnic respiratory failure can be avoided, a higher oxygen dosage is beneficial for COPD patients, e.g. in view of a greater physical ability.

Automated oxygen supply devices capable of automatically adjusting oxygen supply to patients on the basis of oxygen saturation values provided by an oximeter have been suggested, e.g. in US 3,734,091 A. Such automated systems may, in more sophisticated versions, include a closed-loop control wherein oxygen saturation values are read continuously or at least in a higher frequency than in a manual method, and an oxygen supply is adjusted on the basis of these values. As reported by Lellouche, Can. Respir. J. 20, 2013, 259-261, however, closed-loop oxygen titration devices are still not in common use in medicine, neither in hospitals nor in home care treatment.

This may be due to lack of user-friendliness and safety or reliability of systems according to the prior art.

The object of the present invention is therefore to provide improved means for delivering oxygen to patients while preventing hypercapnic respiratory failure.

### Disclosure of the invention

According to the present invention, a system for delivering oxygen to, and for preventing hypercapnic respiratory failure in, a patient, and a corresponding method comprising the features of the independent claims is provided. Preferred embodiments are subject of the dependent claims and of the description that follows.

It should be noted that, if the present application uses the term "oxygen," this term also includes oxygen-rich fluids which do not entirely consist of oxygen. The term "oxygen" according to the language as used herein, therefore, also includes fluids which are enriched in oxygen, "enriched" meaning an oxygen content which is above the oxygen content of atmospheric air, i.e. above 25%, 50% and/or 75% by volume.

If, here and in the following, reference is made to units, modules and devices etc. which are "adapted" to perform a certain function, such units, modules and devices comprise means that are specifically designed, shaped, or operable to perform this function. Such means may be implemented as hardware or software means.

### Advantages of the invention

According to the present invention, a system for delivering oxygen to, and preventing hypercapnic respiratory failure in, a patient, is provided. The system comprises an oxygen dosing unit and a sensor arrangement. The oxygen dosing unit is adapted to provide an oxygen flow to a port which is fluidly connectable to an oxygen delivery unit attached to the patient. The oxygen delivery unit, which may be attached to the port of the oxygen dosing unit by means of suitable tubing and via any kind of coupling devices as known from the prior art, may e.g. comprise a facial mask and/or a nasal cannula, depending on the kind of oxygen treatment that is to be performed. Generally, the present invention may be used in supplemental oxygen treatment methods, e.g. methods including positive airway pressure (PAP) or and variants thereof, e.g. continuous positive airway pressure (CPAP), variable positive airway pressure (VPAP), automatic positive airway pressure (APAP) or bi-level positive airway pressure (BPAP), especially for COPD patients. For details of oxygen delivery units usable in such methods, reference is made to the literature cited above as well as to relevant guidelines for Oxygen Therapy.

The oxygen dosing unit is, according to the present invention, further adapted to regulate parameters of the oxygen flow on the basis of a first input signal indicating an oxygen status of the patient and a second input signal indicating a carbon dioxide status of the patient. In the context of the present invention, "parameters" of the oxygen flow may e.g. include a flow rate, a pressure and an oxygen content of the oxygen flow. As mentioned above, in the context of the present invention the term "oxygen" may also include oxygen-rich fluids which do not consist of oxygen only. Therefore, the oxygen content of such oxygen-rich fluids may be varied, e.g. by admixing air and/or by adjusting operating parameters of an oxygen enrichment device, if such a device is used. An "oxygen status" or a "carbon dioxide status" may, in this context, refer to any value describing, indicating, or being related to, a level or concentration of oxygen or of carbon dioxide in the blood of the patient, especially an arterial saturation value (SaO₂, SaCO₂) or an arterial partial pressure (PaO₂, PaCO₂).

According to the present invention, the sensor arrangement comprises sensing means adapted to obtain a first sensor value corresponding to the oxygen status via a non-invasive transcutaneous measurement and a second sensor value corresponding to the carbon dioxide status via a non-invasive transcutaneous measurement. The sensor arrangement is adapted to provide the first and second input signals on the basis of the first and second sensor values. The present invention, therefore, relies on a transcutaneous measurement especially of the carbon dioxide status in contrast to common end-expiratory measurements.

Pulse oximetry for measuring the SaO₂ by means of pulse oximetry is commonly known as a method of transcutaneous measurement. The principle of this method is based on measuring and evaluating changes in the absorption of light caused by the pulsatile inflow of arterial blood into a well-perfused part of the body (e.g. a finger pad or an ear lobe). The SpO₂ measured in this way normally provides reliable information about the patient's oxygenation. Pulse oximetry is routinely employed in various medical fields, in particular for intra- and postoperative patient monitoring and in homecare treatment.

The most precise way to measure the carbon dioxide status in a patient is to remove and analyse an arterial blood sample. Although this method allows direct measurement of the PaCO₂, it has the disadvantage that it is invasive and requires access to an artery. In addition, the measurement is usually not continuous and therefore does not allow changes in the PaCO₂ to be monitored continuously. The method has the further disadvantage that the analytical result is usually available only after a delay of several minutes. PaCO₂ sensors allowing for continuous and on-time measurement, e.g. integrated in catheters, are invasive, costly, and used exclusively in a controlled hospital environment such as critical or intensive care.

In end-expiratory capnometry, in contrast, an optical absorption measurement in the infrared region is performed in order to determine the concentration of carbon dioxide in the expired gas mixture. The PaCO₂ can be calculated from the carbon dioxide concentration in the end-expiratory phase. However, as an indirect method, capnometry does not always correctly reflect the PaCO₂. It is known that the calculated value is often an underestimate. It is also possible for other parameters, e.g. a change in the cardiac output, to result in a change in the end-expiratory carbon dioxide concentration and thus cause an incorrect estimate of the PaCO₂.

Transcutaneous carbon dioxide measurement, as performed according to the present invention, is likewise indirect and makes e.g. use of the fact that carbon dioxide is able easily to diffuse through body tissue and skin. The gas is measured with a sensor attached to the surface of the skin. When a sensor of this type is warmed to a temperature from about 41 °C to about 45 °C, this produces local dilatation and arterialization of the capillary bed at the measurement site. Under these conditions, the transcutaneous carbon dioxide partial pressure (PtCO₂) measured there shows a good correlation with the arterial value. This makes it possible, with certain restrictions, to determine the PaCO₂ with an accuracy which is sufficient for most applications. Obviously, a corresponding measurement can be calibrated on the basis of an analysis of an arterial blood sample. Instead of measuring carbon dioxide that has diffused through the skin, also optical methods for transcutaneously measuring carbon dioxide may be used according to the present invention, if available.

A "transcutaneous" measurement, therefore, according to the language as used herein, refers to any type of measurement wherein either the analyte (e.g. as carbon dioxide which diffuses through the skin) or a measurement beam (like in pulse oximetry) passes through the skin.

A sensor arrangement that may be used according to the present invention is disclosed in US 6,654,622 B1. Such a sensor arrangement comprises a sensor which has means for pulse oximetric measurement of the arterial oxygen saturation, means for measurement of the transcutaneous carbon dioxide partial pressure, and means for warming a sensor contact surface intended for contact with the ear lobe. The sensor arrangement further comprises means for attaching it to an ear lobe. The means for pulse oximetric measurement of the arterial oxygen saturation include at least two LEDs and one photodiode which are arranged so that when the device is attached to an ear lobe they are located on the same side of the ear lobe, and which are arranged in depressions forming light channels and point towards the sensor contact surface. The means for measurement of the transcutaneous carbon dioxide partial pressure comprise an Ag/AgCl electrode and a glass pH electrode. The means for measurement of the transcutaneous carbon dioxide partial pressure are thus adapted to perform a potentiometric measurement of the carbon dioxide diffused through the skin.

The present invention is, however, not limited to a sensor arrangement which is disclosed in US 6,654,622 B1 but may be used with all types of pulse oximetry sensors and sensors allowing for a transcutaneous measurement of carbon dioxide. It is especially preferred to use sensing means adapted to potentiometrically measure carbon dioxide diffused through the skin of the patient for the transcutaneous measurement of carbon dioxide. Preferentially, these means also include a heating element adapted to heat a localized region of the skin of the patient. Using a corresponding sensor arrangement, measurement precision, reliability and patient comfort may be significantly improved.

However, also an optical measurement of carbon dioxide is envisaged in the context of the present invention. This is preferred because, in contrast to the measurement method as just described, no skin irritations due to heating of the skin are caused.

According to the present invention, the oxygen dosing unit comprises a control unit which is programmed with an algorithm adapted to calculate the parameters of the oxygen flow on the basis of the first and second input values and at least one further patient parameter relating to the patient. In connection with the improved precision of the transcutaneous carbon dioxide measurement, the present invention, including the algorithm as mentioned, results in a much tighter adaption of the oxygen dosage to the needs of the patient, in turn resulting in improved patient comfort and health. With a highly reliable carbon dioxide measurement, parameters of the algorithm may be tuned towards a maximum oxygen dosage not causing hypercapnic respiratory failure, while classic methods need to include much larger safety margins.

The at least one further patient parameter may be a measured or determined value like movement, breathing rate, heart rate, blood pressure, skin temperature or body temperature or it may be a patient status that is entered by the patient or by a clinician like age, sex, body weight, activity status, etc. The at least one further patient parameter, if possible, may also be determined by sensor means that are part of the sensor arrangement as well, thus creating a highly integrated oxygen dosage system.

For example, by taking into account an activity status (the patient may e.g. walk around, sit, or may be asleep), different dosage levels of oxygen may be appropriate. In some cases, e.g. to increase physical ability for a limited period of time, e.g. if the patient intends to climb stairs, the oxygen dosage level may be increased above a regular level which is intended to avoid hypercapnic respiratory failure. In other words, an increase in carbon dioxide during a short time may be accepted while in a long term view oxygen dosage is limited to a level avoiding such a carbon dioxide increase.

Phases of increased or decreased physical activity may e.g. be determined by registering an increased heart rate and/or a increased breathing rate. To differentiate phases of increased activity from other possible causes of an increased heart rate and/or a increased breathing rate like infection and inflammation, a change rate of the heart rate and/or the breathing rate can be evaluated. In phases of increased activity. generally, the heart rate and the breathing rate generally rises comparatively fast while in infection and inflammation, the increase is rather slow. The present invention may include the use of filter functions to eliminate short term increases. To differentiate increases caused by physical activity from normal values for the heart rate and/or the breathing rate, these normal values may be entered manually, e.g. by a physician. However, a normal level can also be acquired using a learning function, e.g. by long-term observation of the heart rate and/or the breathing rate. By observing the heart rate and/or the breathing rate, the system according to the present invention may advantageously also be used as an activity sensor. For example, different activity levels for a specific patient may also be defined, based on the heart rate and/or the breathing rate. In this way, a further dedicated activity sensor may be omitted.

A physical activity may, according to the present invention, also be determined by using a signal of a movement indicator which is included in the system according to the present invention. A movement indicator may include a movement sensor, e.g. an accelerometer. Such an accelerometer may also be part of the sensor arrangement as mentioned above. A corresponding movement indicator may provide an automatic feedback value on the basis of which the control unit may, using the algorithm mentioned above, regulates the oxygen flow. It may also be advantageous to use a location sensor, e.g. a GPS sensor, together with a sensor for the pulse rate and/or the breathing rate, for activity detection. A movement of the patient involving physical activity may in this way be differentiated from cases wherein the patient moves by car, train, etc. A location sensor like GPS is also another safety feature in that in allows localizing the patient in emergencies.

The oxygen dosing unit or the algorithm according to the present invention may, according to a preferred embodiment, select a dosage protocol, e.g. a target oxygen status and/or a target carbon dioxide status, on the basis of the first and second input values and on the basis of the at least one further patient parameter including a patient status as mentioned above, especially the physical activity.

The patient may, e.g. before going to bed, set the patient status to "sleeping," on which basis the oxygen dosing unit then selects an appropriate target oxygen and/or carbon dioxide status. When planning physical activity, this can likewise be communicated to the oxygen dosing unit via the communications interface and the oxygen dosing unit selects an appropriate dosage protocol. An appropriate dosage protocol may also be selected according to an age, a sex, a weight, or a physical condition of the patient.

Patient parameters entered by the patient or the clinician may also include a value indicating the extent as to which the patient "retains" carbon dioxide. From clinical practice, different degrees for the risk of hypercapnic respiratory failure are known, probably resulting from different degrees of the Va/Q mismatch. Some patients therefore, may sooner show a critically increased carbon dioxide status than others. In the former, a more careful dosage of oxygen is required than in the latter. The algorithm used according to the present invention may take this into account. In other words, an individual health condition of the patient is preferentially taken into account.

The oxygen dosing unit is, according to the present invention, preferentially provided as a single device, i.e. it is a constructive unit including several modules which do not need to be separated from each other if the oxygen dosing unit is moved from one place to another, e.g. from one patient to another. Therefore, the oxygen dosing unit according to the present invention is highly user-friendly and reduces the risk of human error, e.g. by wrongly interconnecting such modules. In a particularly preferred embodiment of an oxygen dosing unit according to the present invention, several or all modules mentioned for the oxygen dosing unit in the following may be included in, or permanently attached to, a common housing. This also reduces the risk of damage of an oxygen dosing unit as the housing protects its parts.

According to the present invention, the oxygen dosing unit comprises a communications interface which is adapted to receive input from, or to indicate information to, a user. Such a user interface module may e.g. include a responsive screen which may be used to display and enter information, e.g. patient parameters.

As will be appreciated from the following explanations, a "communications interface" that may be used in an oxygen dosing unit according to the present invention may comprise means for data display and data input that may be presented in physical or virtual form to a user. The communications interface may e.g. include a screen and a keyboard or keypad or a touchscreen displaying information and providing virtual buttons or entry fields to a user. The communications interface may, however, also partially or exclusively be adapted for remote communication only, i.e. it may be a module which is operated remotely, e.g. by an external personal computer or via a local area or wide area data network.

According to a particularly preferred embodiment of the present invention, the control module of the oxygen dosing unit is adapted to store at least one oxygen dosage protocol selectable on the basis of the oxygen dosing data provided to the oxygen dosing unit. An "oxygen dosage protocol" does e.g. include target values for the oxygen and the carbon dioxide status of the patient, i.e. the "first" and "second" values as indicated above, as mentioned.

According to a particularly preferred embodiment of the present invention, the dosing unit or its control module is adapted to store and/or provide history data, the history data including at least one of the parameters of the oxygen flow, the first value, the second value, the oxygen dosing data and an oxygen dosing protocol based thereon, at multiple points of time. To increase privacy, corresponding data, e.g. patient data, may also be stored in encrypted form. The memory module may also be adapted to store a history or protocol of any of the data mentioned above in a form that prevents manipulation, e.g. including hash tags and/or encryption. In this way, the data may be used to document proper patient treatment in case this should be contested.

The communications interface of the oxygen dosing unit according to the present invention may, as mentioned, be adapted for local communication and/or for remote communication with at least one external device. For local communication, as mentioned, e.g. touchscreens, keypads and the like may be used. Remote communication may be established on the basis of wireless communication according to at least one wireless communication protocol. A wireless communication protocol usable according to the present invention may be any protocol as known in the prior art, e.g. WIFI (IEEE 802.11a/b/g/n), WPAN (IEEE 802.15.4) or Bluetooth (IEEE 802.15.1). Modifications of such protocols may also be used, e.g. 6LoWPAN or ZigBee. The wireless communication protocol may also be a proprietary protocol specifically adapted for the purposes of the invention. The wireless communication protocol may include encrypted or unencrypted communication. Wireless communication may also include cellular mobile telephony including corresponding standards.

Especially, also the values relating to the oxygen and the carbon dioxide status of the patient may be wirelessly transmitted from the sensor arrangement to the oxygen dosing unit, the oxygen dosing unit or one of the modules as described in this case being adapted to wirelessly receive corresponding values, i.e. the first and second value. By wireless transmission and reception of such values, cables which can especially in homecare treatment be of hindrance to the patient in that they may be teared off or pose tripping hazards, may be completely omitted. For data transmission, especially the communications protocols specifically adapted for sensor data transmission mentioned above can be used. Also a movement indicator as mentioned above, which is adapted to determine a physical activity or and activity status, may transmit its sensor value accordingly.

An oxygen dosing unit according to the present invention may especially include a valve-integrated pressure regulator or even represent a functionality-enhanced valve-integrated pressure regulator. Valve integrated pressure regulators are known per se. When using gas cylinders, the pressure of the compressed gas, e.g. 200 bar, has to be reduced to a pressure suitable for the need of the patient by means of a pressure regulator. Classically, valve arrangements including at least two separate valves were used to that purpose, including an on/off valve directly connected to the gas cylinder and a flow-regulation valve downstream thereof. In such classical valve arrangements, the pressure of the compressed gas in the gas cylinder and the pressure of the oxygen delivery line can be measured via separate manometers and the gas flow can be adapted accordingly on this basis.

As such valve arrangements are, however, rather bulky and their operation is often considered laborious or at least unintuitive, the valve integrated pressure regulators have become increasingly popular during the recent years. A valve integrated pressure regulator may also comprise the two valves mentioned above, i.e. an on/off valve directly connected to the gas cylinder and a flow regulation valve downstream thereof. Such arrangements are enclosed within the scope of the present invention. These valves are, however, typically integrated together with further valves and/or other components into one compact design and may operable via a single device, e.g. a mechanical handle and/or electronic input means.

WO 2012/164240 A2 discloses a valve integrated pressure regulator comprising a control valve with an aperture for compressed gas and a variable aperture obturator. The aperture obturator is coupled for movement with and by an actuator. To monitor a position of the aperture obturator and, correspondingly, of the opening state of the control valve, a valve position monitor is provided. By monitoring the position of the aperture obturator and of a pressure of the compressed gas in the gas cylinder coupled with the valve integrated pressure regulator, a remaining oxygen supply time can be estimated with high accuracy.

The oxygen dosing unit according to the present invention preferentially includes an, or is attachable to, an oxygen supply unit comprising at least one oxygen container adapted to store liquid or gaseous oxygen or concentration means adapted to provide oxygen by enrichment from air. The oxygen dosing unit according to the present invention may also be configured to be attachable to different kinds of such oxygen supply units, especially via specific coupling modules. The oxygen dosing unit according to the present invention may also be fixedly coupled to an oxygen supply unit, a "fixed" coupling meaning a coupling which is not intended to be uncoupled by a user. For refill with oxygen (in case an oxygen container adapted to store liquid or gaseous oxygen is used), the complete unit can in such cases be sent in to an oxygen supplier. This is particularly advantageous in that no mechanical skills are demanded from a user, e.g. from an elderly patient. However, also the separate replacement of the oxygen container is possible.

According to the present invention, the oxygen supply unit may further be adapted to provide data relating to oxygen supply, e.g. a remaining time of oxygen supply. Such data may be output via the communications interface. Such functionality may include corresponding data being displayed via the communications interface and/or a warning or alarm being issued if oxygen is spent or nearly spent.

The data relating to oxygen supply may, according to a particularly preferred embodiment of the invention, include a remaining oxygen supply time, an expiry date, a container type, a container location, an environmental temperature, an oxygen usage, a time since filling, a rate of oxygen usage, an oxygen pressure, an oxygen temperature, usage data, transportation data, and oxygen remaining in the container. Such data may be determined and/or provided by a container monitoring module that may also be adapted to read information provided with the container, e.g. as a barcode and/or a RFID tag. The remaining oxygen supply time, e.g., may be used to schedule attendance of staff to exchange or refill the oxygen storage and supply unit early enough, if necessary. The remaining oxygen supply time may be determined or estimated on the basis of a pressure of the oxygen remaining in the container and on the basis of a recent or average oxygen consumption.

The elements as mentioned above, especially the valve-integrated pressure regulator and/or the oxygen container, may form part of the system for delivering oxygen to, and for preventing hypercapnic respiratory failure in, a patient according to the present invention. This may also include further elements.

The invention also relates to a method for delivering oxygen to, and for preventing hypercapnic respiratory failure in, a patient, which includes, according to the present invention, that a system as explained above is used in the method. This thus takes profit from the advantages as explained above.

Further advantages of the present invention are explained with reference to the appended drawings which illustrate an embodiment of the present invention.

### Brief Description of the Drawings

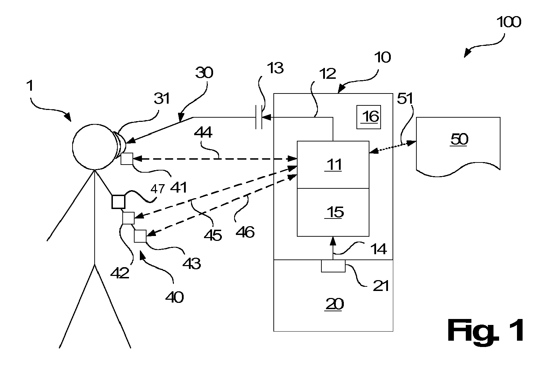

Figure 1 schematically illustrates an oxygen dosing system according to a preferred embodiment of the present invention.

### Embodiment of the Invention

In Figure 1, an system for delivering oxygen to, and for preventing hypercapnic respiratory failure in, a patient according to a preferred embodiment of the present invention is shown and designated 100.

The system 100 as shown in Figure 1 comprises an oxygen dosing unit 10. As a main functional element of the oxygen dosing system 10, a control unit 11 is provided. The control unit 11 is adapted to regulate parameters of an oxygen flow, symbolized 12, which is provided to a port 13 of the oxygen dosing unit 10 as described below.

It will be appreciated by the skilled person that the control unit 11 may be composed of several functional modules or sub-modules, e.g. one or more transmission modules adapted to provide and/or receive, wiredly or wirelessly, signals or data, one or more computation modules adapted to perform computations on data, and/or one or more data storage modules adapted to store data in an appropriate form. Such modules or sub-modules are not shown for clarity.

The oxygen dosing unit 10 according to figure 1 is preferentially provided as a single device, i.e. the modules shown as part of the oxygen dosing unit 10 according to figure 1 may be enclosed or at least partially enclosed in a common housing. The modules shown as part of the oxygen dosing unit 10 according to figure 1 are, at least, mechanically interconnected in a way that allows for the oxygen dosing unit 10 being moved around as a single unit, without a user having to disconnect such modules from each other and/or to transport them separately.

An oxygen supply unit 20 may also be part of such a single device; however, an oxygen supply unit 20 may also be provided separately. For example, the oxygen dosage unit 10 may be provided with an enhanced valve-integrated pressure regulator as described below to which the oxygen supply unit 20 may be appropriately attached, e.g. via couplings known from the prior art.

The oxygen supply unit 20 may comprise means for provision and/or storage of oxygen. Such means may e.g. comprise at least one oxygen container adapted to store oxygen in gaseous or liquid state. Such means may e.g. also comprise oxygen concentration means adapted to provide oxygen by concentrating oxygen from air. Details are omitted for clarity.

As shown in figure 1, the oxygen supply unit 20 comprises an oxygen regulation module 21 which may also be integrated into the oxygen dosing unit 10. The oxygen regulation module 21 may be operated on the basis of a signal from the oxygen dosing unit 10, its control unit 11 or at least one module or sub-module thereof via appropriate signal paths based on wired or wireless connections.

The oxygen regulation module 21 may, in case at least one pressurized oxygen container is used in the oxygen supply unit 20, comprise one or more valves. The oxygen regulation module 21 may also, in case at least one oxygen container for liquid oxygen is used in the oxygen supply unit 20, comprise evaporator means for evaporation of liquid oxygen. In case oxygen concentration means are provided as part of the oxygen supply unit 20, the oxygen regulation module 21 may also regulate one or more functionalities of such oxygen regulation module 21.

A flow of oxygen from the oxygen supply unit 20 or its oxygen regulation module 21 is designated 14. Parameters of the oxygen flow 14 may be the same or different from parameters of the oxygen flow 12 which is provided to the port 13 of the oxygen dosing unit 10, i.e. the parameters of the oxygen flow 14, like its flow-rate, its pressure, or its oxygen content may be further influenced by the oxygen supply unit 20 or its control unit 11 to provide the oxygen flow 12.

The oxygen dosing unit 10 is equipped with a communications interface 15 which may e.g. include a display, especially an interactive display, user input means, especially a keypad, a communications module adapted to communicate with one or more external devices and/or acoustical, optical and/or audio-visual alarm means. The communications interface 15 is adapted to receive input of a user of the oxygen dosing unit 10 or the system 100, e.g. a clinician and/or a patient 1 and is preferentially also adapted to inform a user of a status of the oxygen dosing unit 10 or the system 100 and/or the patient 1. The communications interface 15 is adapted to interact with at least the control unit 11 of the oxygen dosing unit 10.

The port 13 of the oxygen dosing unit 10 is, in the oxygen dosing unit 10 as shown in figure 1, coupled to an oxygen delivery unit 30 via an appropriate coupling module, the coupling module e.g. comprising coupling means adapted to provide any kind of coupling, e.g. screwed or bayonet, to the oxygen dosing unit 10. The oxygen delivery unit 30 further comprises an oxygen supply line extending between the port 13 and a patient module 31. In the example shown, the patient module 31 comprises a facial mask, but any other suitable means of oxygen supply to a patient 1 may be provided in a patient module 31, e.g. a nasal cannula.

The oxygen dosing system 100 as shown in figure 1 further comprises a sensor arrangement 40, the sensor arrangement 40 comprising sensor means 42, 43 adapted to provide a value related to an oxygen status of the patient 1 and a value related to an carbon dioxide status of the patient 1. Corresponding sensor means were described previously. They may especially be included in a single housing. To provide the value related to the oxygen status of the patient 1, a sensor means 42 comprising an oximeter as described above may be provided. The sensor means 43 adapted to provide a value related to a carbon dioxide status of the patient 1 is likewise configured for a transcutaneous measurement. Further sensor means 41 can be provided, e.g. sensor means adapted to measure a heart rate and/or a breathing rate. Furthermore, a motion indicator, e.g. including an accelerometer 47, may be provided.

The sensor means 42, 43 and the accelerometer 47 may be at least partially be integrated into one common module that may be e.g. attached to a suitable part of the body of the patient 1. A common module provided thereby causes minimum hindrance to the patient 1, e.g. in homecare treatment, or to clinical staff which otherwise has to attach several sensor modules 42, 43. Despite shown at an arm of the patient 1 in Figure 1, they likewise can be attached to another body part, e.g. an earlobe.

The sensor arrangement 40 as a whole or its sensor modules 41, 42, 43 are equipped with data transmission means adapted to provide the mentioned values to the oxygen dosing unit 10 via one or more transmission pathways, generally designated 44, 45, 46. The data transmission means of the sensor arrangement 40 as a whole or its sensor modules 41, 42, 43, and likewise corresponding means of the oxygen dosing unit 10, its control module 11 or one or more modules or sub-modules thereof may be adapted for wireless or wired data exchange.

The term "data exchange" primarily relates to a unidirectional data exchange, i.e. a data transmission from the sensor arrangement 40 or one or more of its sensor modules 41, 42, 43 to the oxygen dosing unit 10 or its control module 11, but also to a bidirectional data exchange. In the latter case, the oxygen dosing unit 10 or its control module 11 may e.g. provide one or more signals to the sensor arrangement 40 or one or more of its sensor modules 41, 42, 43 triggering a specific measurement or switching a measurement modality.

The oxygen dosing unit 10 of the system 100 as shown in figure 1 or its control module 11 is adapted to regulate parameters of the oxygen flow 12 on the basis of the first value indicating the oxygen status of the patient 1 and on the basis of the second value indicating the carbon dioxide status of the patient. Such parameters may, as mentioned, include a flow rate, a pressure and/or an oxygen concentration. Therefore, the oxygen dosing unit 10 and the system 100 may provide for titrated oxygen supply, i.e. an oxygen supply which is adapted to the specific oxygen requirements of the patient 1 at each time, without risk for overdosing.

The oxygen dosing unit 10 or its control module 11 may also be adapted to regulate parameters of the oxygen flow 12 on the basis of oxygen dosage data and/or a patient status being provided to the oxygen dosing unit 10 via a communications interface 15. Such oxygen dosage data may be provided by a clinician on the basis of a diagnosis performed and may e.g. include data appropriate to select one or more dosage protocols including e.g. target levels of an oxygen and/or carbon dioxide concentration in the blood of the patient 1. Generally, the control unit 11 may be programmed with an algorithm adapted to calculate the parameters of the oxygen flow 12 on the basis of the first and second input values and at least one further patient parameter relating to the patient 1, as previously explained in detail.

The control module 11 and/or a data storage module thereof may thus also store one or more oxygen dosage protocols e.g. including different target levels of the oxygen and/or carbon dioxide concentration in the blood and/or in the exhaled breath of the patient 1. From these one or more oxygen dosage protocols, optimum values may be selected according to the specific needs.

For example, oxygen dosage data may include several different target levels of the oxygen and/or carbon dioxide concentration as the one or more oxygen dosage protocols, selectable on the basis of further patient information that may be input via the communications interface 15. For example, in different situations, the optimum and/or allowable oxygen and/or carbon dioxide concentrations may also be different. Via the communications interface 15, therefore, the patient 1 and/or a clinician may enter corresponding information, e.g. indicating that the patient 1 is at rest and/or intends to perform physical activity, probably requiring a higher oxygen supply. To avoid any kind of user error, such values can also be solely determined by a movement indicator. Such data thus also relate to dosage data.

Different target levels of the oxygen and/or carbon dioxide concentration may also be selectable from or as the one or more oxygen dosage protocols on the basis of further patient information that may be obtained from further sensor modules of the sensor arrangement 40. Such further information may e.g. include heart rate, blood pressure, blood circulation, breathing rate and breathing volume. On this basis, an optimum oxygen supply, avoiding both hypoxia and hypercapnia, is provided at any time.

A clinician and/or the patient 1 may also change the oxygen dosage data on the basis of a location of the patient, e.g. based on whether the patient is at home, i.e. home care treatment is performed, or whether the patient is in the controlled environment of a clinic, where rapid intervention is possible when a patient status deteriorates. Therefore, based on location data, more or less "conservative," i.e. fail-safe, settings for the oxygen dosage data or for different target levels of the oxygen and/or carbon dioxide concentration may be used.

In order to determine the location of the patient, the oxygen dosing unit 10 may also be equipped with a location module 16, e.g. including a global positioning sensor. The oxygen dosing unit 10 may also include mobile communications means adapted to be communicate via cellular mobile telephony. In this case, a corresponding location module 16 may also determine a location on the basis of a communication cell the mobile communications means are connected with.

The oxygen dosing unit 10 or its control module 11 is, as mentioned, especially adapted to communicate with one or more external devices, e.g. personal computers, hand-held devices and/or mobile phones. The oxygen dosing unit 10 may also be integrated into a local area communications system of a clinic and/or a wide area communication system based on cellular mobile telephony. Communication may especially be performed via the communications interface 15.

Via external devices, therefore, oxygen dosage data may be entered, e.g. via an appropriate computer program or "app" on an external device, and/or a patient status and/or the values provided by the sensor means 41, 42, 43 of the sensor arrangement 40 may be displayed. As mentioned, the oxygen dosing unit 10 or its control module 11 are preferentially adapted to provide an alarm in case a general patient status deteriorates and/or in case the oxygen or carbon dioxide status is out of range, e.g. does not correspond to an expected value for the oxygen dosage data.

Such an alarm may also be output to an external device, e.g. in an emergency office. For example, on the basis of such an alarm, an emergency response may automatically be initiated. In this context, the location module 16 as indicated above may also be used, e.g. informing the emergency office of a location of the patient in case the patient is unable to respond, e.g. in case of unconsciousness.

The oxygen dosing unit 10 or its control module 11 may also include a memory for retrievably storing any one or more of the data as mentioned above, especially a history or protocol of oxygen and/or of the oxygen and/or carbon dioxide status of the patient at several points of time. This memory may e.g. be accessible by the communications interface 15, locally and/or remotely, in order to allow hospital staff to review corresponding data and make treatment decisions based thereon.

Such an arrangement is of particular use when the memory is incorporated in association with the oxygen dosing unit 10 such that it moves together with the oxygen dosing unit 10. This will allow hospital staff to quickly and easily read the data of an incoming or new patient and integrate that data into any patient management or any patient treatment programme. The oxygen dosing unit 10 or its control module 11 may also be operable to transmit any one or more of the data as indicated above to an external device as mentioned. Transmission may also be performed periodically, e.g. every hour or every day, in order to allow for externally backing up such data.

Specifically, a documentation unit 50 is part of the oxygen dosing system 100 according to figure 1. The documentation unit 50 may be wirelessly connected to the oxygen dosing unit 10 and may be adapted to store a history or protocol of the data mentioned. External data storage in the documentation unit 50 may, in order to increase privacy, also be performed in encrypted form.

## Claims

1. A system (100) for delivering oxygen to, and preventing hypercapnic respiratory failure in, a patient (1), the system (100) comprising an oxygen dosing unit (10) and a sensor arrangement (40), the oxygen dosing unit (10) being adapted to provide an oxygen flow (12) to a port (13) fluidly connectable to an oxygen delivery unit (30) attached to the patient (1), and the oxygen dosing unit (10) further being adapted to regulate parameters of the oxygen flow (12) on the basis of a first input signal indicating an oxygen status of the patient (1) and a second input signal indicating a carbon dioxide status of the patient (1) **characterized in that** the sensor arrangement (40) comprises sensing means (42, 43) adapted to obtain a first sensor value corresponding to the oxygen status via a non-invasive transcutaneous measurement and a second sensor value corresponding to the carbon dioxide status via a non-invasive transcutaneous measurement, the sensor arrangement (40) being adapted to provide the first and second input signals on the basis of the first and second sensor values, and the oxygen dosing unit (10) comprising a control unit (11) programmed with an algorithm adapted to calculate the parameters of the oxygen flow (12) on the basis of the first and second input values and at least one further patient parameter relating to the patient (1).

2. A system (100) according to claim 1, wherein the at least one further patient parameter includes an activity status of the patient (1), wherein a movement indicator is provided which is adapted to determine a movement of the patient (1), and wherein the control unit (11) is adapted to determine the activity status of the patient (1) on the basis of a signal of the movement indicator.

3. A system (100) according to claim 1 or 2, the at least one further patient parameter including at least one of a breathing rate, a heart rate, a blood pressure, a skin temperature or a body temperature.

4. A system (100) according to claim 3, the sensor arrangement comprising sensor means (41) adapted to determine the at least one further patient parameter.

5. A system (100) according to any of the preceding claims, the at least one further patient parameter including a patient status including at least one of age, sex, body weight, physical activity and individual health condition.

6. A system (100) according to any of the preceding claims, the control module (11) being adapted to store at least one oxygen dosage protocol selectable by the algorithm on the basis of the first and second input values and on the basis of the at least one further patient parameter.

7. A system (100) according to any of the preceding claims, the sensing means (43) adapted to obtain the second sensor value being adapted to potentiometrically measure carbon dioxide diffused through the skin of the patient.

8. A system (100) according to claim 6, the sensing means (43) adapted to obtain the second sensor value including a heating element adapted to heat a localized region of the skin of the patient (1).

9. A system (100) according to any of the preceding claims, the oxygen dosing unit (10) being embodied as a single unit.

10. A system (100) according to any of the preceding claims, the oxygen dosing unit (10) comprising a communications interface (15) adapted to receive input from, or to indicate information to, a user.

11. The oxygen dosing unit (10) according to claim 10, the communications interface (15) being adapted for local communication and/or for remote communication with at least one external device.

12. An oxygen dosing unit (10) according to any one of the preceding claims, the control module (11) being adapted to store and/or provide history data, the history data including at least one of the parameters of the oxygen flow (12), the first value, the second value and the oxygen dosing data at multiple points of time.

13. The oxygen dosing unit (10) according to any one of the preceding claims, including an, or attachable to, an oxygen supply unit (20) comprising at least one oxygen container adapted to store liquid or gaseous oxygen or concentration means adapted to provide oxygen by enrichment from air.

14. A method of medical oxygen dosing, **characterized in that** an oxygen dosing system (100) according to any one of claims 1 to 10 is used.
